# EUROPEAN PATENT SPECIFICATION

(11) **EP 2 669 364 B1**
(45) Date of publication and mention of the grant of the patent: **19.07.2017**
(21) Application number: 12169684.3
(22) Date of filing: 28.05.2012
(51) Int. Cl.: C12M 1/113

(54) **Biogas reactor**
Biogasreaktor
Réacteur de biogaz

(43) Date of publication of application: 04.12.2013
(73) Proprietor: Energiutvecklarna Norden AB, 590 76 Vreta Kloster (SE)
(72) Inventor: Berg,Lars, 614 30 Söderköping (SE); Carlsson, Jörgen, 616 91 Åby (SE); Andersson, Anders, 611 90 Ålberga (SE); Hakansson, Niklas, 603 75 Norrköping (SE)
(74) Representative: Zacco Sweden AB

(56) References cited:
- WO-A1-84/00378
- WO-A1-2005/113469
- WO-A2-2008/142007
- DE-A1- 19 624 268

## Description

The present invention relates to a biogas reactor according to the preamble of claim 1. The invention also relates to a method of producing biogas according to claim 8.

### PRIOR ART

In the document WO84/00378 A1 a biogas reactor is disclosed that have an agitator and a return flow.

WO 2005/113469 A1 discloses a method for the anaerobic fermentation of biological waste.

DE 196 24 268 A1 discloses a process and assembly for converting especially organic waste into gas for energy conversion.

WO 2008/142007 A2 discloses a fermenter for generating biogas from organic material.

### SHORT DESCRIPTION OF THE INVENTION

The biogas reactor of the prior art lacks means so as to control the gas production as efficient as possible, in particular to start-up. Another problem with the reactor of the prior art is that there is no discussion with regard to possibility to handle high dry solids contents. A further problem with reactor of the prior art is that the agitator is not arranged such that it can aid the production of biogas.

A solution to at least one of the problem is proposed by a biogas reactor according to claim 1, comprising a housing with an inlet for organic material, a biogas outlet, a material displacement device comprising a shaft wherein the biogas reactor has at least two outlets in a gas producing section of the reactor from which organic material can be returned to the inlet.

The advantage of this is that the biogas reactor and can be controlled more precisely by a more advanced grafting. The organic material is slowly transported, from the inlet to the outlet in the biogas reactor and the material broken down biologically and gas is produced in the gas producing section. By giving the possibility of mixing organic material from different outlets it becomes possible to graft the incoming material either with an optimal mixture of organic material and/or micro organisms that will optimize the biogas production. Or by determination depending on the organic material feed to the reactor the optimum outlet for the return feed to the outlet, thus the at least two outlets are adding more flexibility to the biogas reactor, with regard to different organic materials.

The material displacement device comprises a baffle. By introducing a baffle as part of the displacement means there is an obvious advantage that further surface is provided on which the micro organisms/and or organic material can attach for the biogas production process.

The baffle is arranged to have a torsion of 45 - 90° per length of the shaft. This has the effect that the material in the reactor is mainly transported around the shaft, thus primarily not being moved towards the outlet. And also if foam occurs the foam will be pressed downwards at the surface of the organic material (in water suspension), thus reducing any foaming problems. Per length of shaft should be understood as essentially the length of the housing on the inside of the biogas reactor.

In an embodiment according to claim 2 of the biogas reactor the shaft is arranged to be able to provide heat to the biogas reactor. This will further aid the biological break down of the organic material. And it will also aid the possibility to control the biogas reactor and the biogas production by controlling the heating from inside the organic material. Thus the temperature can be controlled from the centre of the housing for an optimized production.

In another embodiment according to claim 3 of the biogas reactor it can be provided with a housing arranged to be able to provide heat to the biogas reactor. By providing heat from the housing to the organic material it is possible to control the biogas reactor and the biogas production.

In another embodiment according to claim 3 both the housing and the shaft can provide heating. This has the effect that the process is possible to control even more as heating in the housing and heating in the shaft can cooperate for an effective and even heating of the organic material. This makes it possible to provide a temperature for the organic material throughout the biogas reactor that is kept within a narrow temperature interval. This further increases the possibility for sanitation of the organic material that is treated/used in the biogas reactor. Thus allowing the biogas reactor to perform the sanitation within the same chamber as where the gas production is performed. Thus avoiding further process steps where sanitation needs to be performed.

In another embodiment according to claim 4 the baffle comprises means for retaining biogas producing micro organisms and/or organic material. By adapting the baffle further the retaining surface can be extended, and thus an even more effective biogas reactor is provided.

In an embodiment of the retaining means according to claim 5 they comprise borings protruding through the baffle, preferably the borings has a diameter within the interval of 0.1 - 10 mm, more preferably in the interval 2 - 4 mm.

The borings are thus intended to retain micro organisms and organic material for a good environment for aid of the biological break down of the organic material.

According to another embodiment according to claim 6, the biogas reactor has a return feed from the at least two outlets mixed in a predetermined ratio.

By determining a optimal ratio between the zones to send back to the inlet, the process can be improved further. The determination and the mixing ratio will depend on such factors as type of organic material and/or other process parameters, such as pH etc.

According to another embodiment according to claim 7, the biogas reactor's material displacement device is arranged to be able to rotate preferably at a speed within the range five rotations per 24 hours - one rotation per 96 hours, preferably 1 rotation per 24 hours.
A slow rotation does not remove the organic material that the micro organisms digest, thus the process is improved. This slow rotation generally would give problems with foaming, but as the biogas reactor is more efficient in particular with regard to the design of the material handling device it can handle this slow rotation.
A method of producing biogas with a biogas reactor according to any of the embodiments above is also provided.

### LIST OF DRAWINGS

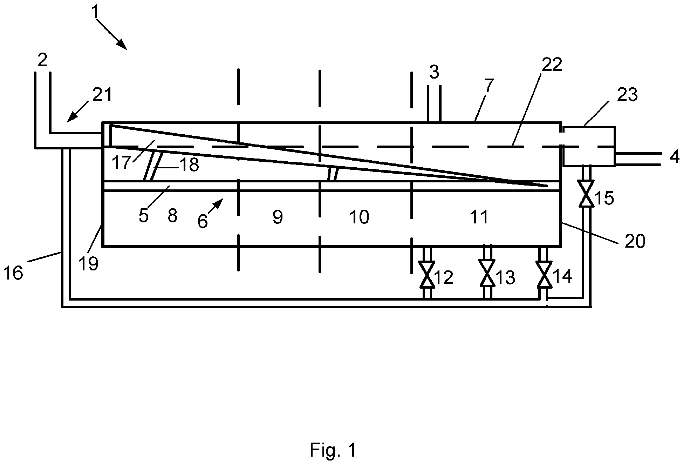

Fig.1 discloses a biogas reactor according to the preferred embodiment.

### DETALIED DESCRIPTION

A preferred embodiment of the biogas reactor will now be described with reference to the accompanying Figure 1.

The biogas reactor has a housing 7 that defines an enclosed chamber that preferably has an essentially horizontal tubular shape. The housing 7 is preferably designed from steel, preferably stainless steel, such as EN 1.4404 (AISI 316L). However other choice of materials is possible, such as aluminium alloys, plastics etc. The shape of the housing 7 is preferably an extended tube with an essentially circular cross section, having a first 19 and a second 20 side wall arranged opposite each other in lateral extension of the tube. However the housing can have other suitable shapes, such as an oval cross section. The longitudinal wall of the housing 7 is generally provided with a heating capability. Also the side walls 19, 20 are preferably heated. That is the wall/walls of the housing 7 can provide heating to the organic material residing inside the housing 7. The heating is generally performed by jacketed heating. This is performed in a manner generally known to the person skilled in the art. Preferably heated water is supplied to the jacket. Other means of heating is also thinkable but they must of course be spark safe and not provide overheating due to the explosive nature of the produced biogas, for example an electrical heating mat would be possible.

The biogas reactor 1 is provided with an inlet 2 for organic material. The inlet 2 is positioned close to one extremity of the housing 7. In a preferred embodiment the inlet is positioned at a distance from the centre of the extremity side wall 19. The inlet 2 is provided with possibility for closing the inlet 2 if needed. The inlet 2 can be provided with heating means 21 for a preheating of the organic material feed. This is particularly advantageous as it gives a quick start of the biological break down process. And also this provides for that when a return feed as described below arrives at the inlet it is not cooled down by the incoming fresh organic material. Preheating also provides for a shorter overall housing as hydrolysis of the organic material can start early.

A gas outlet 3 is suitably provided on the longitudinal wall connecting two side walls 19, 20. The outlet 3 is preferably positioned at the high point of the biogas reactor 1. With high point it should be understood a point that his essentially at the highest point of the biogas reactor housing 7. Should it be necessary for reasons of design to position the gas outlet 3 at another point or if the biogas reactor 1 is inclined the high point can be different from the position of the gas outlet 3. The gas outlet 3 can be closed, if needed.

An organic material outlet 4 is also provided. The outlet 4 is provided at one extremity of the biogas reactor housing 7 on the side wall 20 of this extremity. The outlet 4 can be positioned at any chosen location on this extremity but it is generally positioned at a distance from the centre of the extremity and near the top of this extremity side wall 20. The outlet 4 is provided with a water seal 23. The outlet 4 is feed by organic material by the material displacement device 6. The outlet 4 is preferably situated enclosing the interface of organic material and gas. By having a water seal a simple and effective of the level of organic material in the biogas reactor 1 is achieved. This provided for an emptying of the organic material that is in the final cycle of biological breakdown.

Inscribed in the housing 7 is a material displacement device 6. This device 6 comprises a shaft 5 that is arranged to be able to rotate by means of a drive. The shaft 5 is arranged to be able to provide heat to the organic material residing inside the housing 7 of the bio reactor 1. The heating is in a preferred embodiment provided by means designing the shaft 5 hollow that is provided with heated liquid preferably in the form of water. The shaft 5 is provided with arms 18 that extend to a baffle 17. Preferably the heating is provided by providing a jacket to the shaft 5 such that water only flows in the jacket and not the complete shaft 5.

By providing heating in both the housing 7 and the shaft 5, it is possible to perform sanitation of the organic material in the housing 7 itself, thus it is possible to avoid providing any further chambers , tanks, process steps etc. for sanitation of the organic material. The control of the heating is such that it is possible to perform sanitation of the contents. This is due to the possibility to keep heated throughout the radius of the biogas reactor 1. Sanitation possible to achieve is for example described in Swedish handbook Naturvardsverkets handbook 2003:4. Sanitation can for example by providing 55 °C for 7 days and nights. Other demands, regulations etc, for sanitation are also achievable. Other sanitations could be made by providing; 60 °C for five days; 65 ° for 3 days; 70 °C for 24 hours. Thus there is provided a biogas reactor 1 and a process for producing biogas with the biogas reactor 1 together with sanitation of the organic material such that it can be used for example in agriculture etc, without further sanitation. This is thus in particular possible as the heating is provided from the shaft 5 and the housing 7 as it will be possible to obtain a temperature of the organic material in the biogas reactor 1 that is not varying as it would be if the shaft 5 for example would non heated, thus avoiding a lower temperature next to the shaft 5 compared to the inside wall of the housing 7.

Further the material displacement device is s provided with a baffle 17 that has torsion. The torsion is 45 - 90° per length of the shaft 5. Other torsions are thinkable such as essentially 90° per shaft 5 length. By torsion it should be understood that several essentially non torsioned elements could be attached to eachother at an angle for giving a complete baffle 17 with torsion. The defining factor of the torsion is that it should provide for that the organic material is displaced approximately 1 meter per day away from the inlet 2 towards the outlet 4. A baffle 17 should pass the interface 22 approximately 4 times per 24 hour period. A baffle 17 without torsion is also thinkable preferably combined with another displacement means such that the organic material is transported from the inlet 2 to the outlet 4. The baffle 17 can have three arms 18 of attachment to the shaft 5. However the number of arms 18 is not essential and there could be two to eight arms 18 per baffle. arms. However there cannot be a number of arms 18 that would lessen the open area between the shaft and the baffle 17 to a greater extent, as this would prevent the baffle from displacing the organic material to a large extent. The number of baffles 17 attached to the shaft 5 is preferably four. It is also thinkable to have six or eight baffles, or any suitable number of baffles 17 that provides for a good biogas production. However less than four baffles 17 are not preferred. For reasons of clearness only one baffle has been shown in Fig. 1.

The baffle 17 is in one embodiment provided with retaining means, for retaining organic material. The retaining means is intended to increase the area to which organic matter and micro organisms can attach. The retaining means can comprise blind borings, indentations and/or corrugations. In one preferred embodiment the retaining means comprises borings that extend through said baffle 17. The borings should not be intended to transport organic material from one side of the baffle to the other side. Thus their diameter should not be too large. The preferred range is between 0.1 - 10 mm, larger diameter borings or openings tend to not be able to house organic material. The distance between the borings is 5 mm. This distance can be altered but a 5 mm partition is preferred. The borings are preferably essentially 3 mm in diameter.

The rotation speed for displacement device 6 is preferably one rotation per 24 hour period. This can also be slower of faster. However there should be no complete mixing of the whole content of organic material in the biogas reactor 1 and fast rotation of the displacement device 6 should be avoided. Thus as a general rule five rotations per 24 hour period is the fastest allowed rotation, and rotations down to 1 rotation per 96 hour period. In an alternative embodiment there could be arranged a possibility to allow the shaft to rotate backwards for a shorter time period, in order to handle foaming condition.

As foaming can be handled by means of design of the material handling device the rotation need not be so fast. Often foaming problems is suppressed by increasing rotational speed. But with baffles and/a torsion of 45 - 90° per shaft length an occurring foam will be handled by pressing down the foam at the interface between the organic material and gas.

The bio reactor 1 is provided with several graft outlets 12, 13, 14, 15 for organic material. These outlets 12, 13, 14 and 15 are connected via a pipe 16 to the inlet 2. The graft outlets 12, 13, 14 and 15 are positioned at the section 11 where the gas production is occurring. As can be seen the last outlet 15 is actually positioned in the water seal 23. By providing several outlets 12 - 15 it is possible to provide advanced grafting which can be controlled by providing graft feed from the organic material that is in a break down stage where it is most suitable for grafting. It is also possible to provide a mix of organic material that is in different states of brake down. The graft outlets 12, 13, 14 and 15 are preferably provided with regulation means. These regulation means are preferably as disclosed in the form of vents that makes it possible to adjust the ratio of taken out graft of organic material. The number of graft outlets 12, 13, 14 and 15 are at least two, preferably at least three, most preferably at least four. Other numbers of graft outlets is thinkable depending on the flexibility needed for the bio reactor, in terms of organic material used for the production of biogas, such as five graft outlets or even six or more. The graft organic material helps to start up the process of biological breakdown in the biogas reactor 1. As the rotation of the baffle 17 is slow, there are at all times different stages of biological break down formed in the biogas reactor 1. Thus the biogas reactor 1 provides four different sections where the organic material is in different stages of processing. In Fig. 1 has the for major process sections of the biogas process is referred to as 8, 9, 10 and 11. 8 represents a section where a hydrolysis of the organic material occurs. 9 represents a section where fermentation occurs and 10 represents where anaerobe oxidation occurs and 11 represents the gas producing section, i.e. where the biogas consisting of mainly methane is produced. The boundaries between the described sections 8 - 11 is not exact, they are to be understood to be varying depending on several process parameters as type of organic material, and the heating provided etc.
By providing a biogas reactor 1 with such a complex grafting possibility the biogas production can be optimized very precisely. This means that it is possible from the same amount of organic material to obtain the same amount of gas as in a conventional process but at a faster rate. Also the exchange can be augmented. And no after rotting process is needed. And also there is no need to have the hydrolysis step in a separate chamber. And further the process is continuous. And also by providing the heating from both housing 7 and axis 5 it is possible to perform sanitation in the housing 7 of the bioreactor itself.

Generally the process of producing biogas by means of the biogas reactor 1 can be described as this:
Organic material such as manure, effluents or offall is provided to the biogas reactor through the inlet 2. The organic material is heated by the shaft 5 and the jacket of the housing 7 of the biogas reactor 1. If a heating means 21, in the form of a preheater 21, is installed on the inlet 2, heating is provided before entry into the biogas reactor 1. The baffle 17 attached to the shaft 5 slowly rotates the material around the shaft and organic material is retained in the borings of the baffle 17. The biological breakdown process is started and gas is produced by the micro organisms that break down the organic material. The produced gas rises to the top of the housing 7 and is allowed to exit through the gas outlet 3. The organic material in a first section 8 of the housing 7 undergoes hydrolysis and after slow transport the biological material enters the second section 9 where fermentation takes place. When the organic material enters the third section zone 10 the organic material undergoes mainly anaerobe oxidation. And as the fourth section 11 is approached the gas production starts. In the final section of section 11 the break down is slower as more and more of the organic material is broken down and the micro organisms start to be in excess of organic material that can be broken down. The break down and consequently the production of biogas are accelerated as the number of micro organisms is increased by propagation. The baffles 17 finally lift the organic material to the outlet 4 where it is allowed to exit. During the transport through the housing 7 a minor part of the organic material is taken out of one or several of the graft outlets 12, 13, 14 , 15 and feed back to the inlet 2 for grafting the incoming organic material. The mixing ratio of the outlets or the single outlet from which grafting is to be performed is predetermined by empirical testing depending on the organic material used, for an optimized grafting at the inlet 2 of the biogas reactor 1.

By the construction of the biogas reactor 1 in particular with regard to the baffle 17 and the slow rotation rate of the displacement device 6, the dry solids in the biogas reactor can be relatively high compared to known reactors. A dry solids content of 10 % or even up to 14 % - 15 % is possible for this type of reactor. The advantage of this is evident as with less water in the organic material, the heating of it will demand less energy, and the production of biogas can be optimized further. As stated above the process is faster and all the steps of the biogas production process i.e. hydrolysis, fermentation, anaerobe oxidation and methane production, can be performed in the same chamber.

The wording biological breakdown should be understood to embody all the biological processes that transform organic materials such that biogas is generated. In particular it should be construed as thermophilic rotting.

The wording organic material is to be understood any material that can be turned into biogas in a process by micro organisms. Thus also in addition to manure, effluents or offal, any waste from agriculture, foresting and any other suitable material is to be construed as organic material, in terms of the described and claimed biogas reactor and method, also industrial waste etc.

The biogas reactor 1 in one embodiment has the dimensions such that it can be fitted into a standard container. Thus the biogas reactor 1 can then easily be moved if desired.

Further is described method for producing biogas using a biogas reactor 1 according to any design above
comprising the steps of
a. providing organic material to the biogas reactor 1
b. heating the organic material in the biogas reactor 1
c. rotating the organic material by means of a material displacement device 6
d. allowing the organic material thereby to undergo hydrolysis, fermentation, anaerobe oxidation and gas production,
whereby grafting of the incoming organic material is performed from a predetermined grafting outlet (12, 13, 14, 15), wherein the biogas reactor allows the choice between at least two grafting outlets.

In a further development the method step b comprises that the heating provided to the organic material is such that sanitation of the organic material is provided.

## Claims

1. Biogas reactor (1) comprising a housing (7) with an inlet (2) for organic material, a biogas outlet (3), a material displacement device (6) comprising a shaft (5) **characterized in that** the biogas reactor (1) has at least two outlets (12, 13) in a gas producing section (10) of the reactor (1) from which organic material can be returned to the inlet (2), and wherein the material displacement device (6) comprises a baffle (17), wherein the baffle (17) is arranged to have a torsion of 45 - 90° per length of the shaft (5).

2. Biogas reactor (1) according to claim 1, wherein the shaft (5) is arranged to be able to provide heat to the biogas reactor (1).

3. Biogas reactor (1) according to claim 1 or 2, wherein the housing (7) is arranged to be able to provide heat to the biogas reactor (1).

4. Biogas reactor (1) according to claim 1, wherein the baffle (17) comprises means for retaining biogas producing micro organisms and/or organic material.

5. Biogas reactor (1) according to claim 4, wherein the retaining means comprises borings protruding through the baffle (17), preferably the borings has a diameter within the interval of 0.1 - 10 mm, more preferably in the interval 2 - 4 mm.

6. Biogas reactor (1) according to any of the claims above, wherein a return feed from the at least two outlets (12, 13) is mixed in a predetermined ratio.

7. Biogas reactor (1) according to any of the claims above, wherein the shaft (5) and thus the material displacement device (6) is arranged to be able to rotate preferably at a speed within the range five rotations per 24 hours - one rotation per 96 hours, preferably one rotation per 24 hours.

8. Method for producing biogas using a biogas reactor 1 according to any of the claims 1 - 7 comprising the steps of
a. providing organic material to the biogas reactor 1
b. heating the organic material in the biogas reactor 1
c. rotating the organic material by means of a material displacement device 6
d. allowing the organic material thereby to undergo hydrolysis, fermentation, anaerobe oxidation and gas production,
whereby grafting of the incoming organic material is performed from a predetermined grafting outlet (12, 13, 14, 15), wherein the biogas reactor allows the choice between at least two grafting outlets.

9. Method for producing biogas according to claim 8, where in step b comprises that the heating provided to the organic material is such that sanitation of the organic material is provided.

## Patentansprüche

1. Biogasreaktor (1) umfassend ein Gehäuse (7) mit einem Eingang (2) für organisches Material, einen Biogasausgang (3), eine Materialversatzeinrichtung (6) umfassend einen Schaft (5), **dadurch gekennzeichnet, dass** der Biogasreaktor (1) mindestens zwei Ausgänge (12, 13) in einem Gaserzeugungsabschnitt (10) des Reaktors (1) aufweist, von welchen organisches Material in den Eingang (2) zurückgeführt werden kann, und wobei die Materialversatzeinrichtung (6) ein Leitblech (17) umfasst, wobei das Leitblech (17) ausgebildet ist, um eine Torsion von 45 - 90° je Länge des Schafts (5) aufzuweisen.

2. Biogasreaktor (1) nach Anspruch 1, wobei der Schaft (5) ausgebildet ist, um Wärme an den Biogasreaktor (1) liefern zu können.

3. Biogasreaktor (1) nach Anspruch 1 oder 2, wobei das Gehäuse (7) ausgebildet ist, um Wärme an den Biogasreaktor (1) liefern zu können.

4. Biogasreaktor (1) nach Anspruch 1, wobei das Leitblech (17) Mittel zum Zurückbehalten von Biogas erzeugenden Mikroorganismen und/oder organischem Material umfasst.

5. Biogasreaktor (1) nach Anspruch 4, wobei die Zurückbehaltungsmittel Bohrungen aufweisen, die durch das Leitblech (17) hindurch vorspringen, wobei die Bohrungen vorzugsweise einen Durchmesser im Bereich von 0,1 - 10 mm, eher bevorzugt im Bereich von 2 - 4 mm, aufweisen.

6. Biogasreaktor (1) nach einem der vorgehenden Ansprüche, wobei eine Rückspeisung von den mindestens zwei Ausgängen (12, 13) in einem vorgegebenen Verhältnis gemischt wird.

7. Biogasreaktor (1) nach einem der vorgehenden Ansprüche, wobei der Schaft (5) und somit die Materialversatzeinrichtung (6) ausgebildet ist, um vorzugsweise bei einer Geschwindigkeit innerhalb des Bereichs von fünf Rotationen je 24 Stunden bis eine Rotation je 96 Stunden, vorzugsweise eine Rotation je 24 Stunden, rotieren zu können.

8. Verfahren zur Erzeugung von Biogas unter Verwendung eines Biogasreaktor 1 nach einem der Ansprüche 1-7 umfassend die folgenden Schritte
a. Bereitstellen von organischem Material für den Biogasreaktor 1
b. Erhitzen des organischen Materials in dem Biogasreaktor 1
c. Rotieren des organischen Materials mittels einer Materialversatzeinrichtung 6
d. Veranlassen des organischen Materials dazu, einer Hydrolyse, einer Fermentation, einer anaeroben Oxidation und einer Gasherstellung unterworfen zu werden,
wobei die Pfropfung des eingehenden organischen Materials ausgehend von einem vorgegebenen Pfropfungsausgang (12, 13, 14, 15) durchgeführt wird, wobei der Biogasreaktor die Wahl zwischen mindestens zwei Pfropfungsausgängen erlaubt.

9. Verfahren zur Erzeugung von Biogas nach Anspruch 8, wobei im Schritt b enthalten ist, dass die dem organischen Material bereitgestellte Wärme einer solchen Art ist, dass eine Reinigung des organischen Materials vorgesehen ist.

## Revendications

1. Réacteur de biogaz (1) comprenant un boîtier (7) avec une entrée (2) pour une matière organique, une sortie de biogaz (3), un dispositif de déplacement de matière (6) comprenant un arbre (5), **caractérisé en ce que** le réacteur de biogaz (1) présente au moins deux sorties (12, 13) dans une section de production de gaz (10) du réacteur (1) à partir de laquelle une matière organique peut être renvoyée à l'entrée (2), et dans lequel le dispositif de déplacement de matière (6) comprend un déflecteur (17), ledit déflecteur (17) étant agencé pour avoir une torsion de 45 à 90 ° par longueur de l'arbre (5).

2. Réacteur de biogaz (1) selon la revendication 1, dans lequel l'arbre (5) est agencé pour pouvoir fournir du chauffage au réacteur de biogaz (1).

3. Réacteur de biogaz (1) selon la revendication 1 ou 2, dans lequel le boîtier (7) est agencé pour pouvoir fournir du chauffage au réacteur de biogaz (1).

4. Réacteur de biogaz (1) selon la revendication 1, dans lequel le déflecteur (17) comprend des moyens pour retenir des microorganismes et/ou une matière organique produisant du biogaz.

5. Réacteur de biogaz (1) selon la revendication 4, dans lequel le moyen de retenue comprend des perçages faisant saillie à travers le déflecteur (17), de préférence les perçages présentant un diamètre dans l'intervalle de 0,1 à 10 mm, plus préférablement dans l'intervalle de 2 à 4 mm.

6. Réacteur de biogaz (1) selon l'une quelconque des revendications précédentes, dans lequel une alimentation de retour provenant des au moins deux sorties (12, 13) est mélangée dans un rapport prédéterminé.

7. Réacteur de biogaz (1) selon l'une quelconque des revendications précédentes, dans lequel l'arbre (5) et ainsi le dispositif de déplacement de matière (6) sont agencés pour pouvoir tourner de préférence à une vitesse dans la plage de cinq rotations par 24 heures - d'une rotation par 96 heures, de préférence d'une rotation par 24 heures.

8. Procédé de production de biogaz à l'aide d'un réacteur à biogaz (1) selon l'une quelconque des revendications 1 à 7, comprenant les étapes consistant à
a. fournir une matière organique au réacteur de biogaz (1)
b. chauffer la matière organique dans le réacteur à biogaz (1)
c. faire tourner la matière organique au moyen d'un dispositif de déplacement de matière (6)
d. permettre à la matière organique ainsi de subir une hydrolyse, une fermentation, une oxydation d'anaérobie et une production de gaz,
le greffage de la matière organique entrante étant effectué à partir d'une sortie de greffe prédéterminée (12, 13, 14, 15), le réacteur de biogaz permettant le choix entre au moins deux sorties de greffage.

9. Procédé de production de biogaz selon la revendication 8, dans lequel l'étape b comprend que le chauffage fourni à la matière organique est tel que l'assainissement de la matière organique est prévu.
